(19) Europäisches Patentamt
European Patent Office
Office européen des brevets

(11) Veröffentlichungsnummer: **0 215 136**
**B1**

(12) # EUROPÄISCHE PATENTSCHRIFT

(45) Veröffentlichungstag der Patentschrift: **22.06.88**

(51) Int. Cl.⁴: **A 61 K 31/505,** A 61 K 9/18, A 61 K 47/00

(21) Anmeldenummer: **85111020.5**

(22) Anmeldetag: **02.09.85**

(54) **Hexetidin 1,3-Bis-(2-ethylhexyl)-hexahydro-5-methyl-5-pyrimidinamin enthaltendes therapeutisch wirkendes Mittel, sowie Verfahren zum Herstellen desselben und Verwendung als vaginales Desinfektionsmittel.**

(43) Veröffentlichungstag der Anmeldung:
**25.03.87 Patentblatt 87/13**

(45) Bekanntmachung des Hinweises auf die Patenterteilung:
**22.06.88 Patentblatt 88/25**

(84) Benannte Vertragsstaaten:
**AT BE CH DE FR GB IT LI NL SE**

(56) Entgegenhaltungen:
**LU - A - 76 238**

(73) Patentinhaber: **Artesan Pharma Gesellschaft mit beschränkter Haftung, Wendlandstrasse 1, D-3130 Lüchow (DE)**

(72) Erfinder: **Hotzel, Knut A., Wendlandstrasse 1b, D-3130 Lüchow (DE)**
Erfinder: **Peterssen-Borstel, Hartwig, Dipl.-Chem. Dr., Steine Nr. 4, D-3131 Luckau (DE)**

(74) Vertreter: **von Raffay, Vincenz, Dipl.-Ing. et al, Patentanwälte Raffay, Fleck & Partner Postfach 32 32 17, D-2000 Hamburg 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Hexetidin (1) enthaltendes therapeutisch wirkendes Mittel, ein Verfahren zum Herstellen desselben und seine Verwendung.

(1) HEXETIDIN
Hexetidinum

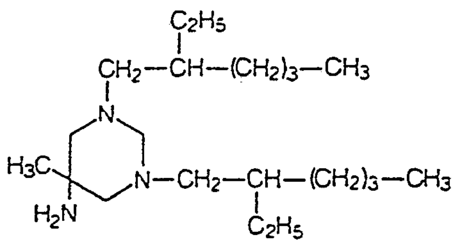

1,3-Bis-(2-ethylhexyl)-hexahydro-5-methyl-5-pyrimidinamin

Hexetidin ist ein Antisepticum, das stark wirksam ist und dabei ein breites Wirkungsspektrum gegen gram-positive und gram-negative Bakterien hat. Der grösste Vorzug der Substanz ist die Tatsache, dass sie seit Jahrzehnten in Form einer 0,1%igen Lösung sowohl als therapeutisches als auch kosmetisches Gurgelwasser eine grosse Verbreitung gefunden hat.

Geht man davon aus, dass auf der Schleimhaut beim Gurgeln nur wenig resorbiert wird, während im wesentlichen nur eine Adsorption stattfindet, so muss man doch annehmen, dass ein bedeutender Teil der gelösten Substanz verschluckt und über den Magen-Darm-Trakt aufgenommen wird. Eine toxische Wirkung von Hexetidin ist dabei trotz der grossen Verbreitung und der Verwendung über 20 Jahre nie beobachtet worden.

Hexetidin ist eine unangenehm fischig riechende Flüssigkeit. Wie ersichtlich, ist Hexetidin chemisch ein Triamin mit zwei tertiären und einer primären Aminogruppe. Aufgrund des Überwiegens aliphatischer Reste ist Hexetidin in Wasser nur schwer löslich, leicht löslich dagegen ist es in Alkohol, aber auch in unpolaren organischen Lösungsmitteln. Um Hexetidin in wässerige Lösung zu bekommen, hat man durch Zufügung von Säure das Hexetidinsalz gebildet, welches dann in Wasser leicht löslich ist. Die andere Möglichkeit ist der Einsatz von Emulgatoren.

Aufgrund über Jahre geführter Untersuchungen sind die bekannten Methoden nicht geeignet, ein stabiles pharmazeutisches Präparat herzustellen. Hexetidin ist in neutraler bis schwach saurer wässeriger Lösung nur kurzfristig haltbar. Es lagert sich bis zu einem Gleichgewicht in zwei isomere Substanzen um. Zwar wirken diese im Prinzip gleichartig, jedoch dürfen sie aus gesundheitsrechtlichen Gründen im Endprodukt nur geringfügig vorhanden sein.

Zusammenfassend kann man sagen, dass es sich nachteilig bemerkbar gemacht hat, dass der Wirkstoff Hexetidin unter den meisten Bedingungen instabil und nur auf ein allzu eng begrenztes therapeutisches Gebiet gerichtet ist.

Der vorliegenden Erfindung liegt deshalb die Aufgabe zugrunde, ein Hexetidin enthaltendes therapeutisch wirkendes Mittel zu schaffen, das unter den üblichen Bedingungen stabil ist und in seiner Handhabung leicht und einfach ist. Ein weiteres Ziel der vorliegenden Erfindung liegt darin, ein Verfahren zum Herstellen dieses stabilen Hexetidin enthaltenden therapeutisch wirkenden Mittels zu schaffen, das nicht nur wirtschaftlich ist, sondern auch in seinem Ablauf unkompliziert ist. Wenn möglich, sollte eine neue Verwendungsmöglichkeit für den Wirkstoff Hexetidin geschaffen werden, insbesondere im Zusammenhang mit einer neuen galenischen Darreichungsform.

Diese Aufgaben und Ziele werden durch das Mittel gemäss Anspruch 1 und durch das Verfahren zu seiner Herstellung gemäss Anspruch 6 gelöst, während Anspruch 11 den neuen Verwendungszweck angibt.

Die erfindungsgemässe Festarzneizubereitung von Hexetidin bildet also zunächst ein stabiles Adsorbat von Wirkstoff Hexetidin und Lösungsmittel auf einem Träger, wobei gerade diejenigen Bedingungen vermieden werden, die zur Instabilität des Hexetidin führen, nämlich ein wässeriges oder verdünnt alkoholisches Medium, sowie saure Bedingungen oder erhöhte Temperaturen über 40 bzw. 50° C. Neben dem Wirkstoff werden Myristylalkohol und Sorbit eingesetzt, um ein erstes Granulat zu erzeugen. Ggf. können weitere Hilfsstoffe hinzugefügt werden. Das zweite Granulat bzw. Pulver umfasst hingegen die wasserlöslichen und sauren Bestandteile Sorbit und Weinsäure und ggf. Aerosil sowie weitere Spreng- und Tablettiermittel. Das erste und zweite Granulat bildet zusammen im verpressten Zustand die fertige Tablette. Es ist jedoch ebenfalls erfindungsgemäss an eine galenische Zäpfchenform des Wirkstoffs Hexetidin gedacht, das dann bekannterweise unpolare Wachse und Fette umfasst, die anstelle des zweiten Granulats eingesetzt werden, und ebenfalls eine stabile Festarzneizubereitung von Hexetidin ermöglichen.

Myristylalkohol wird eingesetzt, da er äusserst unpolar und bei Raumtemperatur fest ist, während er oberhalb von 37° C verflüssigt werden kann und dann leicht emulgierbar ist. Erfindungsgemäss sind insofern höhere Temperaturen als 37 bzw. 40° C nicht erforderlich.

Die Einbettung bzw. Verpressung der beiden Granulate besitzt den Vorteil, dass die Presslinge bzw. Zäpfchen einen sauren pH-Wert besitzen, nämlich etwa von 4,5 wie das Vaginalsekret selbst. Die Presslinge haben ausserdem den Vorteil, dass sie gut zerfallen und somit die wirkstoffhaltige Phase, nämlich das Granulat I, gleichmässig verteilt wird. Der beim Auflösen entstehende saure pH-Wert lässt eine günstige Verteilung des Hexetidins zwischen organischer und wässeriger Phase zu.

Versuche haben gezeigt, dass das Hexetidin in der erfindungsgemässen Zubereitung für die therapeutisch notwendigen Zeiträume in der Vagina stabil ist. Praktisch wird in etwa eine gleich gut bakterizide Wirksamkeit erzielt wie mit dem im

Handel erhältlichen Jodpräparat Betaisadona. Die erfindungsgemässe Zubereitung ist insofern jedoch fortschrittlicher, da sie den Nachteil des Jods (Resorption einer toxischen Substanz und die Verfärbung von Wäsche und Haut) vermeidet.

Weitere Vorteile und Merkmale gehen aus den Unteransprüchen hervor, auf die hiermit ausdrücklich Bezug genommen wird.

Im folgenden wir ein bevorzugtes Ausführungsbeispiel für die Hexetidin enthaltende therapeutisch wirkende Zubereitung in detaillierter Weise beschrieben, ohne dass die Erfindung hierauf beschränkt ist.

*Beispiel :*

Herstellungsmenge: 17 kg für 20 000 Vaginaltabletten

| *Wirkstoff* | *Pharmakopoe* | *Menge* |
|---|---|---|
| Hexetidin | DAC 79 | 220 g |
| *Hilfsstoffe* | | |
| Cellulose | DAB 8 | 1 400 g |
| Magnesiumstearat | Ph. Eur. III | 180 g |
| Myristylalkohol | Spezifikation | 1 000 g |
| Polyoxyethylen-(20)sorbitanmonostearat | BP 80 | 400 g |
| Poly(1-vinyl-2-pyrrolidon) 25 000 | BP 80 Add. 82 | 100 g |
| Unlösliches Poly-(1-vinyl-2-pyrrolidon) | DAC 79 | 400 g |
| Hochdisperses Siliciumdioxid | DAB 8 | 1 400 g |
| Sorbit | DAB 8 | 11 620 g |
| Triglycerid-Diglycerid- und Monoglyceridgemisch (73:12:15) gradzahliger Fettsäuren($C_{12}$-$C_{18}$) | Spezifikation | 180 g |
| Weinsäure | Ph. Eur. III | 100 g |
| | | 17 000 g |

*Hilfsstoffe, die im Endprodukt nicht mehr enthalten sind*

| | | |
|---|---|---|
| Isopropanol | BP 80 | 900 g |
| Gereinigtes Wasser | Ph. Eur. I | 100 g |

Ein Beispiel für das erfindungsgemässe Herstellungsverfahren wird im folgenden geschildert, wobei die Mengenangaben für ca. 20 000 Vaginaltabletten gedacht sind:

*Granulierung*

*Granulat I*

Eine Lösung von
Hexetidin 220 g
in geschmolzenem Myristylalkohol (40° C) 1000 g
wird warm (40° C) in
Sorbit 2000 g
eingerührt.

Aerosil (hochdisperse, pyrogene Kieselsäure von über 99,8% $SiO_2$) 600 g
und
Elcema G 250 (mikrofeines Cellulosepulver als Tablettier- und Dragiermittel Pudergrundlage) 400 g
werden fein gesiebt (Maschenweite 1,0 mm) und in einem Bäckerkneter vorgelegt.
Unter ständiger Knetbewegung gibt man portionsweise das Hexetidin-Myristylalkohol-Sorbit-Adsorbat zu.
Knetzeit: 15 Minuten

*Granulat II*

| | |
|---|---|
| Sorbit | 7820 g |
| Weinsäure | 100 g |
| Aerosil | 400 g |

werden fein gesiebt (Maschenweite 1,0 mm) und im Bäckerkneter mit der Granulierflüssigkeit durchfeuchtet und 15 Minuten lang durchgeknetet.
Granulierflüssigkeit: Tween 60 400 g
(Polyoxyethylen(20)sorbitanmonostearat)
Kollidon 25 100 g
(Poly(1-vinyl-2-pyrrolidon 25 000)
werden in 1000 g 90%igem (G/G) Isopropanol warm gelöst.

Die Mischung wird durch ein grobes Sieb (Maschenweite 2,5 mm) gegeben und dünn auf Blechen verteilt. Sie wird bei maximal 38° C 8 Stunden lang bei intensiver Belüftung getrocknet.

*Mischung*

| | |
|---|---|
| Granulat I | 4220 g |
| Granulat II | 8820 g |
| Sorbit | 1800 g |
| Polyplasdone (unlösliches Poly(1-vinyl-2-pyrrolidon)) | 400 g |
| Elcema G 250 | 1000 g |
| Aerosil (hochdisperses Siliciumdioxid) | 400 g |
| Boeson | 180 g |

Triglycerid-Diglycerid- und Monoglyceridgemisch (73:12:15) gradzahliger Fettsäuren ($C_{12}$-$C_{18}$) werden fein gesiebt (1,0 mm Maschenweite) und im Taumelfass 8 Minuten gemischt. Zum Schluss wird das Granulat im Taumelfass 2 Minuten lang mit
Magnesiumstearat 180 g
gemischt.

| | Sollgewicht | Toleranzgrenzen |
|---|---|---|
| Chargengewicht | 17,0 | 16,5-17,2 kg. |

Die nachfolgenden Ausführungen eines Versuchsberichtes belegen, dass das erfindungsgemässe Mittel und Verfahren sich zur Behandlung vaginaler Antisepsis in der Schwangerschaft, insbesondere beim Blasensprung, hervorragend einsetzen lassen.

Zu den häufigstens begünstigenden Faktoren eines Amnioninfektionssyndroms (AIS) gehört der vorzeitige Blasensprung (VBLS). Doch ist die Keimbesiedlung von Eihäuten, Plazenta und Fruchtwasser nach heutigen Erkenntnissen auch bei stehender Blase gar nicht so selten, besonders bei vorzeitiger Wehentätigkeit und Cervixinsuffizienz.

Hexetidin besitzt bekanntermassen neben seinen antiseptischen Eigenschaften auch eine geringe Toxizität bei Resorption. Ausserdem besteht eine gute Haut- und Schleimhaut-Verträglichkeit. Es wurden bakteriologische Kontrollen von Scheide und Cervix vor und nach Hexetidin-Vaginaleinlage durchgeführt. Abb. 1 zeigt bei 23 Schwangeren das Verhalten der Döderlein-Flora vor und 20 Stunden nach einmaliger Einlage von ca. 10 mg Hexetidin-Wirkstoff in der erfindungsgemässen Tablette. Sie wird reduziert, jedoch nicht vollständig zerstört. Dieses hängt vom Vorhandensein der Dichte der Laktobazillen vor der Behandlung ab. In einigen Fällen kam es sogar zur leichten Verbesserung, wahrscheinlich infolge der Reduktion von pathogenen Keimen. Die in den Vorkontrollen gefundenen pathogenen Keime waren nach Hexetidin-Einlage entweder reduziert oder nicht mehr nachweisbar (Abb. 2). Dabei benötigten Pilze eine längere Behandlungsdauer.

Abb. 1

*Döderlein-Flora*
Vor und ($\bar{x}$ = 20 h) nach einmaliger Hexetidin-Vaginaleinlage (10 mg)

| N | vor \ nach | +++ | ++ | + | (+) | ∅ |
|---|---|---|---|---|---|---|
| 2 | +++ | X | 1 | | 1 | |
| 7 | ++ | | 2 | 2 | 2 | 1 |
| 11 | + | | 1 | 6 | 1 | 3 |
| 2 | (+) | | | | X | 2 |
| 1 | ∅ | | | | 1 | X |

Σ 23 Schwangere, $\bar{x}$ = 31. Wo

Abb. 2

*Pathogene Scheidenflora*
Vor und ($\bar{x}$ = 20 h) nach einmaliger Hexetidin-Vaginaleinlage (10 mg)
(23 Schwangere, $\bar{x}$ = 31. Wo)

| | N | Gleich | Reduz. | ∅ | Err.-Wechsel |
|---|---|---|---|---|---|
| Aerobier | 8 | 1 | 4 | 3 | 2 |
| Anaerobier | 2 | — | — | 2 | — |
| Pilze | 2 | — | 2 | — | — |

**Patentansprüche**

1. Hexetidin (1,3-Bis-(2-ethylhexyl)-hexahydro-5-methyl-5-pyrimidinamin) enthaltendes therapeutisch wirkendes Mittel, gekennzeichnet durch ein stabiles Adsorbat von Hexetidin mit Myristylalkohol und Sorbit, das in feiner Verteilung in einer wasserlöslichen, sauren Pulver- oder Granulatmischung, umfassend Sorbit, Weinsäure und ggf. weitere Hilfsstoffe, eingebettet und/oder verpresst ist.

2. Hexetidin (1,3-Bis-(2-ethylhexyl)-hexahydro-5-methyl-5-pyrimidinamin) enthaltendes therapeutisch wirkendes Mittel nach Anspruch 2, dadurch gekennzeichnet, dass das Adsorbat als Hilfsstoffe hochdisperses Siliciumdioxid (Aerosil®) und Cellulosepulver umfasst.

3. Hexetidin (1,3-Bis-(2-ethylhexyl)-hexahydro-5-methyl-5-pyrimidinamin) enthaltendes therapeutisch wirkendes Mittel nach Anspruch 1 und 2, dadurch gekennzeichnet, dass die Pulver- oder Granulatmischung als weiteren Hilfsstoff hochdisperses Siliciumdioxid (Aerosil®) umfasst.

4. Hexetidin (1,3-Bis-(2-ethylhexyl)-hexahydro-5-methyl-5-pyrimidinamin) enthaltendes therapeutisch wirkendes Mittel nach Anspruch 1 bis 3, dadurch gekennzeichnet, dass die Pulver- oder Granulatmischung als Sprengmittel unlösliches PVP (Polyvinylpyrrolidon) bzw. lösliches PVP als Granuliermittel aufweist.

5. Hexetidin (1,3-Bis-(2-ethylhexyl)-hexahydro-5-methyl-5-pyrimidinamin) enthaltendes therapeutisch wirkendes Mittel nach Anspruch 1 bis 4, gekennzeichnet durch einen oder mehrere der folgenden Hilfsstoffe:
unlösliches Poly(1-vinyl-2-pyrrolidon) (Polyplasdone®)
Triglycerid-, Diglycerid- und Monoglyceridgemisch (73:12:15) gradzahliger Fettsäuren ($C_{12}$-$C_{18}$)
(Boeson VP®)
Magnesiumstearat
Polyoxyethylen(20)sorbitanmonostearat (Tween 60®)
hochdisperses Siliciumdioxid.

6. Verfahren zum Herstellen von Hexetidin (1,3-Bis-(2-ethylhexyl)-hexahydro-5-methyl-5-pyrimidinamin) enthaltendem therapeutisch wirkenden Mittel nach Anspruch 1 bis 5, gekennzeichnet durch die folgenden Stufen:
a. Zubereiten einer stabilen Lösung von Hexetidin in Myristylalkohol;
b. Einrühren derselben in Sorbit unter Ausbildung eines Adsorbats;
c. Herstellen einer Pulver- bzw. Granulatmischung von Sorbit, Weinsäure und ggf. weiteren Hilfsstoffen;
d. Vermischen des in Stufe b. hergestellten Adsorbats mit der Pulver- bzw. Granulatmischung ggf. unter Zusatz weiterer Hilfsstoffe.

7. Verfahren nach Anspruch 6, dadurch gekennzeichnet, dass in Stufe a. eine 40° C warme Lösung von Hexetidin in Myristylalkohol zubereitet wird.

8. Verfahren nach Anspruch 6 und 7, dadurch gekennzeichnet, dass eine etwa 40° C warme Lösung von Hexetidin in geschmolzenem Myristylalkohol in Sorbit eingerührt wird, und dass Aerosil, sowie mikrofeines Cellulosepulver als Tablettier- bzw. Dragiermittel in einer Knetvorrichtung vorgelegt werden, in die portionsweise unter ständiger Knetbewegung das Hexetidin-Myristylalkohol-Sorbit-Adsorbat unter Ausbildung eines Granulats I zugegeben wird.

9. Verfahren nach Anspruch 6 bis 8, dadurch gekennzeichnet, dass in der Stufe c. Sorbit, Weinsäure und Aerosil mit einer Granulierflüssigkeit durchfeuchtet und durchgeknetet und anschliessend bei leicht erhöhter Temperatur bis maximal 38° C mehrere Stunden unter Erzeugung eines Granulats II getrocknet werden.

10. Verfahren nach einem oder mehreren der vorstehenden Ansprüche 6 bis 9, dadurch gekennzeichnet, dass die Granulate I und II mit weiteren Hilfsstoffen fein gesiebt und miteinander vermischt werden.

11. Verwendung von Hexetidin für die Herstellung eines vaginalen Desinfektionsmittels, insbesondere in Tabletten- oder Zäpfchenform.

## Claims

1. Hexetidine (1,3-bis-(2-ethylhexyl)-hexahydro-5-methyl-5-pyrimidinamine) – containing therapeutically active product, characterized by a stable adsorbate of hexetidine with myristyl alcohol and sorbitol which, in fine distribution, is embedded and/or compressed in a water-soluble, acid powder or granular mixture, comprising sorbitol, tartaric acid and optionally further adjuvants.

2. Hexetidine (1,3-bis-(2-ethylhexyl)-hexahydro-5-methyl-5-pyrimidinamine) – containing therapeutically active product according to claim 1, characterized in that the adsorbate comprises as adjuvants highly disperse silicon dioxide (Aerosil) and cellulose powder.

3. Hexetidine (1,3-bis-(2-ethylhexyl)-hexahydro-5-methyl-5-pyrimidinamine) – containing therapeutically active product according to claims 1 and 2, characterized in that the powder or granular mixture comprises as a further adjuvant highly disperse silicon dioxide (Aerosil).

4. Hexetidine (1,3-bis-(2-ethylhexyl)-hexahydro-5-methyl-5-pyrimidinamine) – containing therapeutically active product according to claims 1 to 3, characterized in that the powder or granular mixture has as the disintegrating agent insoluble PVP (polyvinylpyrrolidone) or soluble PVP as the granulating agent.

5. Hexetidine (1,3-bis-(2-ethylhexyl)-hexahydro-5-methyl-5-pyrimidinamine) – containing therapeutically active product according to claims 1 to 4, characterized by one or more of the following adjuvants:
insoluble polyvinylpyrrolidone (Polyplasdone),
triglyceride-diglyceride and monoglyceride mixture (73:12:15) of even-number fatty acids ($C_{12}$-$C_{18}$) (Boeson VP),
magnesium stearate,
polyoxyethylene(20)sorbitan-monostearate (Tween 60) and
highly disperse silicon dioxide.

6. Process for the preparation of hexetidine (1,3-bis-(2-ethylhexyl)-hexahydro-5-methyl-5-pyrimidinamine) – containing therapeutically active product according to claims 1 to 5, characterized by the following stages:
a. preparation of a stable solution of hexetidine in myristyl alcohol,
b. stirring the same into sorbitol, accompanied by the formation of an adsorbate,
c. preparation of a powder or granular mixture of sorbitol, tartaric acid and optionally further adjuvants,
d. mixing the adsorbate prepared in stage b. with the powder or granular mixture, optionally accompanied by the addition of further adjuvants.

7. Process according to claim 6, characterized in that in stage a. a solution of hexetidine in myristyl alcohol at 40° C is prepared.

8. Process according to claims 6 and 7, characterized in that a solution of hexetidine in molten myristyl alcohol at approximately 40° C is stirred into sorbitol and that Aerosil, as well as microfine cellulose powder as the tabletting and/or coating agent are placed in a kneader, to which is added portionwise and accompanied by a constant kneading movement the hexetidine-myristyl alcohol-sorbitol adsorbate, whilst forming a granular material I.

9. Process according to claims 6 to 8, characterized in that in stage c. sorbitol, tartaric acid and Aerosil are thoroughly moistened with a granulating liquid and thoroughly kneaded, followed by drying at a slightly elevated temperature of up to max. 38° C for several hours, accompanied by the production of granular material II.

10. Process according to one or more of the preceding claims 6 to 9, characterized in that granular materials I and II are finely screened with further adjuvants and mixed together.

11. Use of hexetidine for producing a vaginal disinfectant, particularly in tablet or suppository form.

## Revendications

1. Remède à action thérapeutique à base d'hexétidine (1,3-bis-(2-éthylhexyl)-hexahydro-5-methyl-5-pyrimidinamin), caractérisé par un adsorbat stable d'hexétidine avec de l'alcool de myristyl et du sorbitol qui est inclus et/ou comprimé en étant réparti finement dans un mélange de granulat ou de poudre acide, soluble dans l'eau, comprenant du sorbitol, de l'acide tartrique et éventuellement d'autres auxiliaires.

2. Remède à action thérapeutique à base d'hexétidine (1,3-bis-(2-éthylhexyl)-hexahydro-5-méthyl-5-pyrimidinamine) selon la revendication 1, caractérisé en ce que l'adsorbat contient du dioxyde de silicium bien dispersé (Aérosil®) et de la poudre de cellulose comme auxiliaires.

3. Remède à action thérapeutique à base d'hexétidine (1,3-bis-(2-éthylhexyl)-hexahydro-5-méthyl-5-pyrimidinamine) selon les revendications 1 et 2, caractérisé en ce que le mélange de poudre ou de granulat contient du dioxyde de silicium (Aérosil®) bien dispersé comme autre auxiliaire.

4. Remède à action thérapeutique à base d'hexétidine (1,3-bis-(2-éthylhexyl)-hexahydro-5-méthyl-5-pyrimidinamine) selon les revendications 1 à 3, caractérisé en ce que le mélange de poudre ou de granulat présente du polyvinylpyrrolidone insoluble comme explosif ou du polyvinylpyrrolidone soluble comme agent de granulation.

5. Remède à action thérapeutique à base d'hexétidine (1,3-bis-(2-éthylhexyl)-hexahydro-5-méthyl-5-pyrimidinamine) selon les revendications 1 à 4, caractérisé par un ou plusieurs des auxiliaires suivants:
poly(1-vinyl-2-pyrrolidone) insoluble (Polyplasdone®)
mélange de triglycéride, diglycéride et monoglycéride (73:12:15) d'acides gras d'ordre pair ($C_{12}$-$C_{18}$) (Boeson VP®)
stéarate de magnésium
monostéarate de polyoxyéthylène(20)sorbitane (Tween 60®)
dioxyde de silicium bien dispersé.

6. Procédé pour la fabrication d'un remède à action thérapeutique à base d'hexétidine (1,3-bis-(2-éthylhexyl)-hexahydro-5-méthyl-5-pyrimidinamine) selon les revendications 1 à 5, caractérisé par les phases suivantes:
a. préparation d'une solution stable d'hexétidine dans de l'alcool de myristyl;
b. mélange de celle-ci dans du sorbitol et formation d'un adsorbat;
c. préparation d'un mélange de poudre ou de granulat de sorbitol, acide tartrique et d'autres auxiliaires éventuels;
d. mélange de l'adsorbat fabriqué à la phase b avec le mélange de poudre ou de granulat, éventuellement en ajoutant d'autres auxiliaires.

7. Procédé selon la revendication 6, caractérisé en ce que, pour la phase a, une solution chaude à 40° C d'hexétidine est préparée dans de l'alcool de myristyl.

8. Procédé selon les revendications 6 et 7, caractérisé en ce qu'une solution chaude d'environ 40° C d'hexétidine dans de l'alcool de myristyl fondu est mélangée à du sorbitol et que l'on place de l'Aérosil ainsi que de la poudre de cellulose microfine comme agent pour fabrication de comprimés ou de dragées dans un dispositif malaxeur dans lequel on ajoute l'adsorbat d'hexétidine-alcool de myristyl-sorbitol par portions en malaxant constamment pour former un granulat I.

9. Procédé selon les revendications 6 à 8, caractérisé en ce que, dans la phase c, on humidifie le sorbitol, l'acide tartrique et l'Aérosil avec un liquide de granulation et qu'on le pétrit pour le sécher ensuite pendant plusieurs heures à une température légèrement élevée de 38° C maximum pour former un granulat II.

10. Procédé selon une ou plusieurs des revendications précédentes 6 à 9, caractérisé en ce que les granulats I et II sont tamisés finement avec d'autres auxiliaires et mélangés l'un à l'autre.

11. Utilisation de l'hexétidine pour la fabrication d'un désinfectant vaginal, en particulier sous forme de comprimés ou de suppositoires.